# EUROPEAN PATENT APPLICATION

(11) **EP 4 609 888 A1**
(43) Date of publication of application: **03.09.2025**
(21) Application number: 24160202.8
(22) Date of filing: 28.02.2024
(51) Int. Cl.: A61M 1/06, A61M 1/00

(54) **PUMP SYSTEM FOR BREAST PUMP, BREAST PUMP SYSTEM, METHODS FOR EXTRACTING MILK**

(71) Applicant: Befemtech, 1030 Schaerbeek (BE)
(72) Inventor: DERYCKE, Jerome, 1030 Brussels (BE); LUYCKX, Anastasia, 1200 Brussels (BE); OTMANE TOLBA, Yasmine, 1090 Jette (BE); MADDEN, Philip, 2930 Brasschaat (BE); LUST, Thomas, 2800 Mechelen (BE); JANTON, Hugo, 1300 Wavre (BE); VANDENBUSSCHE, Tom, 1030 Schaerbeek (BE)
(74) Representative: Calysta NV

(57) **Abstract**

Pump system for a breast pump comprising a stepper motor; and a control unit configured to receive user input settings, wherein the user input settings relate to a frequency, an amplitude, and a pressure shape chosen from a plurality of predetermined expression mode pressure shapes; determine an expression mode pressure profile based on the user input settings, wherein for a first expression mode pressure profile based on user settings with a first pressure shape, and a second expression mode pressure profile based on user settings with a second pressure shape, the pressure at the air outlet in function of time differ from each other in more and/or other ways than being scaled in amplitude and/or time.

## Description

The present invention relates to the field of breast pumps for extracting milk from a nipple, in particular from a human breast. The present invention relates in particular to pump systems, breast pump systems, cloud-based infrastructure, methods, and computer-readable instructions.

For newborn babies and young children, the breast milk produced by their mother is a great source of nutrition that provides a wide variety of advantages, both short term and long term. However, for various reasons it may be difficult to directly transfer the milk from the breast of the mother to the child. This may e.g. be the case because the milk production of the mother is not optimal yet (e.g. when the baby is born premature), or the child is not successful yet in extracting the milk from the breast. Another common situation is that the mother is not able to be in the presence of the child at all times, e.g. because she starts working again, but still wishes to feed the child with breast milk. In those situations, a breast pump can be used to extract the milk from the breast. The milk is stored to be fed to the child at a later moment.

Many different breast pumps are available. Typically, the breast pump subjects the nipple to a (usually periodic) pressure profile that varies in function of time, which is meant to emulate to sucking motion of the child. In most commercially available breast pumps, the pressure profile is mainly determined by the type of breast pump being used. Especially with portable pumps, often a vacuum pump is used that releases the pressure very quickly.

Some available pumps allow the user to change some settings, usually the frequency (i.e. length of one period) and the maximal pressure difference. For example, during the stimulation phase a higher frequency is used, and once the milk flow is activated the frequency is reduced during an extraction or expression phase. Some conventional systems also allow the user to adjust the frequency or maximal pressure during the stimulation phase or the extraction/expression phase. However, the shape of the pressure profile in the known systems remains the same, since the pressure profile is only for being scaled in time and/or amplitude.

It is an object of the invention to overcome the disadvantages of the prior art, or at least provide an alternative to the prior art. It is in particular an object of the invention to provide a pump system that allows the user more options in (the shape of) the pressure profile, in particular with a portable breast pump.

This object is achieved with a pump system for a breast pump for extracting milk from a nipple of a human breast, the pump system comprising,
- at least one pressure outlet comprising at least one outlet membrane, wherein the pressure outlet is configured to be fluidly connected to a breast cup configured to be arranged on the breast (e.g. on/over the nipple),
- a force unit configured to move the at least one outlet membrane for controlling the pressure at the air outlet, wherein optionally the force unit comprises a stepper motor configured to be moved in steps, and configured to transmit a movement to the outlet membrane with each step

Optionally, the pump system comprises a control unit configured to control the force unit, wherein the control unit is configured to
- receive user input settings, wherein the user input settings relate to a frequency, an amplitude, and a pressure shape chosen from a plurality of predetermined expression mode pressure shapes,
- determine an expression mode pressure profile based on the user input settings, and control the force unit based on the expression mode pressure profile,

In embodiments, for a first expression mode pressure profile based on user settings with a first pressure shape, and a second expression mode pressure profile based on user settings with a second pressure shape, the pressure at the air outlet in function of time differ from each other in more and/or other ways than being scaled in amplitude and/or time.

In embodiments, the control unit is configured to determine a step sequence for the stepper motor based on the expression mode pressure profile.

For example, the first expression mode pressure profile and the second expression mode pressure profile can both be based on user settings with a first frequency and a first amplitude (thus being the same in for first and second expression mode profile); while the user settings on which the first expression mode pressure profile is based comprised a first pressure shape and the user settings on which the second expression mode pressure profile is based comprised a second pressure shape (being different from the first pressure shape, and both chosen from the plurality of predetermined expression mode pressure shapes). In such embodiments, for the first expression mode pressure profile the control unit is configured determine a first step sequence for moving the stepper motor from a begin step to a first maximal step and back within a first time period, and for the second expression mode pressure profile the control unit is configured to determine a second step sequence for moving the stepper motor from the begin step to the first maximal step and back within the first time period. Optionally, the first step sequence differs from the second step sequence in the size of the steps, and/or the speed in which steps are taken, and/or a time period between subsequent steps, and/or a direction of each of the steps.

The invention thus relates, in embodiments, to a pump system for a breast pump. The breast pump can be used for extracting milk from a human (female) breast. The pump system can e.g. be functionally connected to a breast cup. The breast cup may comprise a nipple receiving portion being configured to be arranged on or around the nipple of the breast. The breast cup may be configured to subject the nipple to a varying pressure, wherein the pump system is configured to generate said varying pressure. The breast cup may e.g. comprise a diaphragm that is configured to move for subjecting the nipple to said varying pressure. The diaphragm may be fluidly connected to the pump system, e.g. via a tube, wherein pressure variations in said fluid connection cause the diaphragm to move. The pump system may be configured to generate said pressure variations in said fluid connections. The diaphragm may be configured to seal an area around the nipple, together with the nipple receiving portion of the breast cup.

The pump system comprises at least one pressure outlet. The pressure outlet is fluidly connected to the breast cup. This allows the pump system to transmit pressure variations from the pressure outlet to the breast cup. The breast cup in turn can transmit pressure variations to the nipple for extracting milk. The fluidic connection between the pressure outlet and the breast cup can e.g. be achieved by a tube, e.g. made of a flexible material, e.g. a polymer. Air can be trapped in the tube to transmit pressure variations. However, it may also be possible that the pressure outlet is directly connected (e.g. without tube) to the breast cup, e.g. when the pump system and breast cup are integrated with each other.

The pressure outlet can e.g. comprise at least one outlet membrane. The outlet membrane can e.g. fluidly close the pressure outlet on a pump side. The outlet membrane can be configured to be moveable. For example, the outlet membrane can be made of a material that can be deformed, e.g. under the applied pressures. The outlet membrane can be made of a flexible material, e.g. a polymer. The moving (e.g. by deforming) of the outlet membrane, generates pressure variations in the fluidic connection to the breast cup.

In some embodiments, the pump system can be used for extracting milk from a single human breast. In these embodiments, it may be possible that the pressure outlet comprises a single outlet membrane.

In some embodiments, the pump system can be used for extracting milk from multiple human breasts at the same time, e.g. from both breast of the same mother. In these embodiments, it may be possible that the pressure outlet comprises a single outlet membrane that is fluidly connected to two breast cups. For example, the pressure outlet may comprise a first fluidic connection to a first breast cup and a second fluidic connection to a second breast cup, wherein movement of the single outlet membrane is configured to cause pressure variations in both fluidic connections. It may be possible that both the first and second fluidic connection are connected to a separate pressure outlet connector, or it may be possible that a single fluidic connection branches of into the first and second fluidic connection (e.g. embodied by a Y-shaped tubing). However, it is also possible that the pressure outlet comprises a first outlet membrane fluidly connected to the first breast cup, and a second outlet membrane fluidly connected to the second breast cup.

The pump system further comprises a force unit. The force unit is configured to generate/cause (and control) the pressure variations at the pressure outlet and as such the pressure variations that are transmitted to the breast cup. In particular, the force unit can be configured to move the at least one outlet membrane. By controlling the pressure variations, e.g. by means of the outlet membrane, the force unit enables the pumping function of the pump system. The force unit may e.g. include a drive system, e.g. an (electric) motor.

Optionally, the force unit comprises a stepper motor. A stepper motor is a motor that moves in steps. With each step, a movement is transmitted to the outlet membrane. The steps can e.g. be angular steps, which can e.g. be converted to linear motion which is transmitted to the at least one outlet membrane. The stepper motor can e.g. be electrically driven, e.g. by an electric motor, e.g. a brushless and/or DC motor.

The use of a stepper motor in the pump system may be particularly advantageous. It can provide a relatively compact drive system that allows for high precision and controllability. As explained in more detail further below, the breast pump can be controlled according to different settings, e.g. having different expression mode pressure shapes, frequencies, and/or amplitudes. A stepper motor allows this flexibility. In addition, the stepper motor can be used while producing very little noise. This can be advantageous, e.g. using quiet settings when the mother uses the system next to a sleeping baby or e.g. at the office. In other situations noise can be less annoying, and then the mother can choose settings which are optimized to extract as much milk as quickly as possible. In addition, as explained in more detail below, the stepper motor allows a compact design of the pump system which can be portable during use.

In embodiments, the stepper motor is the main drive system of the force unit. In embodiments, the stepper motor is the only drive system of the force unit, and/or the pump system.

Optionally, the pump system further comprises a control unit. The control unit is configured to control at least the force unit. Thus, the control unit can control the movement of the force unit, and as such the pressure variations (e.g. because the movement of the outlet membrane is controlled) at the pressure outlet that are transmitted to the breast cup. As such, the control unit is configured to control the pressure variations to which the nipple is subjected.

Optionally, the control unit is configured to control the force unit, e.g. in accordance with a pressure profile. During a stimulation mode, the pressure profile may be a stimulation mode pressure profile. During an expression mode, the pressure profile may be an expression mode pressure profile. The pressure profile represents the pressure that the nipple is subjected to. Usually, the nipple is subjected to substantially periodic pressure variations, which is reflected in the pressure profile. The pressure profile can be characterized by a frequency (reflecting the length of a period), a maximal pressure difference (reflecting the maximal negative pressure to which the nipple is subjected), and a pressure shape. The pressure shape reflects how the pressure evolves from a begin pressure to a maximal negative pressure and back, e.g. within one period. Examples of pressure shapes are sinusoidal, polynomial, a combination of polynomial shapes and/or sinusoidal shapes, a sequence of polynomial shapes, a combination of sinusoidal shapes with different frequency, or a sequence of sinusoidal with different frequency and amplitude. The pressure shape generally reflects the pressure (variations) to which the nipple is exposed. Thus, the pressure shape relates to the pressure (variations) which are generated at the breast cup, e.g. in the nipple receiving portions, e.g. by the diaphragm of the breast cup, which in turn is e.g. controlled via the pressure outlet of the breast pump. To verify a pressure shape, it may be possible to provide a pressure sensor in the breast receiving portion, e.g. instead of a nipple when using the breast pump in a test environment.

Optionally, the control unit is configured to receive user input settings. User input settings are input by a user of the pump system. This may e.g. be the mother who is applying the pump system to extract milk from here breast, but in some cases it the user may also be a professional (e.g. nurse, midwife, or doctor) helping the mother. The user input settings relate to a desired pressure profile. In particular, the user settings can relate to the frequency and/or the amplitude and/or the pressure shape of the pressure profile. Although generally the user settings will include information based on which the control unit is configured to determine the frequency, amplitude, and pressure shape, it is not necessary that the user selects all three separately. A single option to be selected by the user may e.g. reflect two or more of the frequency, amplitude, and pressure shape. It is also possible that the user does explicitly selects the frequency, amplitude, or pressure shape, but that this is derived from inputs of the user. For example, the user may input factors such as: how old the baby is; any relevant medical issues (e.g. sore nipples); the time of day.

Optionally, the pressure shape (as being part of the user input settings) is chosen from a plurality of predetermined expression mode pressure shapes. Thus, the user can choose between different expression mode pressure shapes. The pressure shapes can e.g. include one or more of: sinusoidal, polynomial, a combination sequence of polynomial shapes and/or sinusoidal shapes, a sequence of polynomial shapes, a combination of sinusoidal shapes with different frequency, or a sequence of sinusoidal with different frequency and amplitude.

Being expression mode pressure shapes, said pressure shapes are to be applied in a pressure profile that is used during an expression mode. Generally a breast pump first applies a stimulation mode. Similar to the sucking behavior of the child, the stimulation mode applies a high-frequency pressure profile. This stimulates the breast to start the flow of milk. Once the flow is going, the breast pump (as does the child) will shift to a lower frequency pressure profile. This is the expression mode. Thus, it is during this expression mode, that the control unit allows the user to choose between different pressure shapes. However, similar consideration may apply in addition or alternatively to the stimulation mode. It is also possible that the control unit is configured to apply the same pressure shape for the expression mode and the stimulation mode.

The different pressure shapes are different compared to each other, even if the frequency and amplitude would be equal. Thus, the different pressure shapes are not just scaled version of each other.

In particular, the control unit is configured to determine an expression mode pressure profile based on the user input settings. For a first expression mode pressure profile based on user settings with a first pressure shape, and a second expression mode pressure profile based on user settings with a second pressure shape, the pressure at the air outlet in function of time differ from each other in more and/or other ways than being scaled in amplitude and/or time.

In these embodiments, control unit thus allows the user to choose an expression mode pressure shape. This is not possible with conventional (portable) breast pumps, which are limited to a single pressure shape (especially when keeping frequency and amplitude constant). However, it has been found that some mothers react better to certain pressure while other mothers react better to other shapes. In addition, the needs of a mother can evolve over time as she goes through her breastfeeding journey. For example, at some moments she may encounter nipple pain and require a pressure shape that is softer, while at other moments she may want to increase her production and require a pressure shape that she finds work best for her in terms of milk extraction. This is a big hurdle in practice, because breast pumps can be a large investment (in a time period where young parents usually already have many expenses). With these embodiments, the risk of buying a breast pump which does not function optimally for the particular mother are reduced significantly.

Based on the expression mode pressure profile, the control unit controls the force unit. For example, the control unit controls when the force unit takes which steps, and how fast said step is taken. The control unit is e.g. configured to determine a step sequence for the stepper motor based on the expression mode pressure profile.

It will be understood that many different expression mode pressure profiles are possible when the amplitude, frequency, and pressure shape can be selected in the user settings. A particular feature of these embodiments relates to a first and second expression mode pressure profile with the same frequency and amplitude, but different pressure shape. That is, for a first expression mode pressure profile with a first frequency, a first amplitude and a first pressure shape; the control unit is configured to move the stepper motor in a first step sequence from a begin step to a first maximal step and back within a first time period. For a second expression mode pressure profile with the first frequency, the first amplitude and a second pressure shape, the control unit is configured to move the stepper motor in a second step sequence from the begin step to the first maximal step and back within the first time period,

The first step sequence differs from the second step sequence in the size of the steps, and/or the speed in which steps are taken, and/or a time period between subsequent steps, and/or the direction of each step. For example, the first and second step sequence differ from in each other in the size of the steps, and the speed in which steps are taken, and a time period between subsequent steps For example, the first and second step sequence differ from in each other in the size of the steps, and the speed in which steps are taken. For example, the first and second step sequence differ from in each other in the size of the steps, a time period between subsequent steps. For example, the first and second step sequence differ from in each other in the speed in which steps are taken, and a time period between subsequent steps.

By applying different step sequences, different shapes of the pressure profile are possible.

Optionally, when the control unit if configured to determine the expression mode pressure profile based on the user input settings, the control unit is configured to determine the expression mode pressure profile as a periodic function. Optionally, the length of a period of the periodic function is based on the frequency. Optionally, a minimum and/or maximum of the periodic function is based on the amplitude. Optionally, the shape of the periodic function is based on the pressure shape.

Optionally, the control unit is configured to determine a step sequence based on an expression mode pressure profile. For example, the control unit is configured to determine the first step sequence based on the first expression mode pressure profile, and/or the second step sequence based on the second expression mode pressure profile. For example, the expression mode pressure profile may represent the desired pressure profile at the nipple or at the pressure outlet. The control unit may apply a transfer function to determine the step sequence. For example, the transfer function may reflect how steps of the stepper motor affect the pressure at the nipple or the pressure outlet, and/or the (inverted) transfer function may reflect how the pressure at the nipple or the pressure outlet are affected by steps of the stepper motor. The transfer function can e.g. be determined theoretically, e.g. based on how the stepper motor is connected to the outlet membrane(s). It may also be possible that the transfer function is determined empirically, e.g. by measuring the respective pressure (changes) caused by the steps of the stepper motor in a test environment.

Based on the expression pressure mode profile, and e.g. the (inverted) transfer function, the control unit may be configured to determine a desired movement of the stepper motor. Based on the desired movement, the control unit may be configured to determine the step sequence. It may be possible that the desired movement is a continuous movement, while the stepper motor is configured to move in discrete steps.

Optionally, the control unit is configured to receive adjusted user settings during use. Optionally, the control unit is configured to enable, within each expression mode, to adjust the frequency and/or the amplitude. For example, the adjusted user settings can relate to an adjusted frequency and/or an adjusted amplitude. Thus, the user can adjust the frequency or amplitude during use, e.g. because the pressure difference is too large. The control unit may be configured to determine, based on the adjusted user settings, an adjusted expression mode pressure profile with an adjusted frequency and/or adjusted amplitude and/or an adjusted pressure shape, although it may optionally be possible to keep the same pressure shape. The control unit can e.g. be configured to determine an adjusted step sequence.

In embodiments, (a motor driver of) the stepper motor is configured to take a plurality of different step sizes. For example, the stepper motor can be able to take at least two different step sizes, or at least three different step sizes, or at least four different step sizes. For example, the stepper motor can be configured to take full steps and micro steps, wherein micro steps are smaller than a full step. A full step can e.g. contain up to 256 micro steps. The size of the steps can e.g. be controlled by a motor driver, which may e.g. be incorporated (functionally) in the stepper motor or in the control unit. Optionally, when the stepper motor can take three or more different step sizes, at least three of the step sizes are multiples of each other. Optionally, when the stepper motor can take three or more different step sizes, at least three of the step sizes are not multiples of each other. Optionally, the stepper motor is configured to move with a single step size within a single pressure profile. Thus, when a pressure profile is selected, the step size remains the same.

In embodiments, the plurality of predetermined expression mode pressure shapes comprises at least four different expression mode pressure shapes, each configured to result in mutually different step sequences at equal amplitude and frequency.

In embodiments, at least one of the plurality of predetermined expression mode pressure shapes, e.g. the first pressure shape, is a sinusoidal shape.

In embodiments, at least one of the plurality of predetermined expression mode pressure shapes, e.g. the second pressure shape, is a shape determined by a polynomial having at least five factors, e.g. eight factors.

In embodiments, at least one of the plurality of predetermined expression mode pressure shapes is a combination of polynomial shapes.

In embodiments, at least one of the plurality of predetermined expression mode pressure shapes is a combination of sinusoidal shapes.

In embodiments, at least one of the plurality of predetermined expression mode pressure shapes is a combination of polynomial shapes.

In embodiments, at least one of the plurality of predetermined expression mode pressure shapes is a combination of sinusoidal shape(s) and polynomial shape(s).

In embodiments, at least one of the plurality of predetermined expression mode pressure shapes is a sequence of polynomial shapes.

In embodiments, at least one of the plurality of predetermined expression mode pressure shapes is a sequence of sinusoidal shapes.

In embodiments the first pressure shape has a pressure build-up section and a pressure let-down section, wherein for example:
- the pressure let-down section occupies at least 40% of the first pressure shape in function of time; and/or
- wherein the slope of the pressure let-down section is equal to the slope of the pressure build-up section; and/or
- wherein the pressure build-up section and the pressure let-down section are mirrored to each other relative to a mirror line which extends through a peak.

In embodiments, at least one of the plurality of predetermined expression mode pressure shapes has a pressure build-up section and a pressure let-down section, wherein the pressure let-down section has a first part and a second part, with different slope. In the first part, pressure is released slower than in the second part. Optionally, in the second part, the slope is similar in absolute terms to the slope in the rising part. Optionally, the pressure let-down section occupies more than 50% of the period (the pressure shape in time) in this example.

In embodiments, at least one of the plurality of predetermined expression mode pressure shapes has a pressure build-up section and a pressure let-down section. The pressure let-down section has a steep slope as the pressure is released quickly. The pressure build-up section has a slope that is less steep, as pressure is built up gradually. The pressure build-up section occupies the majority of the period (the pressure shape in time), e.g. more than 80%.

In embodiments, at least one of the plurality of predetermined expression mode pressure shapes has a non-periodic function having a periods, and amplitudes that vary. When a user would select such pressure profile in the user settings, the chosen amplitude and frequency may be the average maximal amplitude and average frequency, respectively. Each single wave has a period; an amplitude, a pressure build-up section , and a pressure let-down section. For a single wave the wave is approximately mirrored around the moment where the amplitude has been reached. Thus, the slope of the respective pressure build-up section s is similar to the slope of the respective pressure let-down section.

In embodiments, at least one of the plurality of predetermined expression mode pressure shapes has a pressure build-up section and a pressure let-down section. For a single period the wave is approximately mirrored around the moment where the amplitude has been reached. Thus, the slope of the pressure build-up section is similar to the slope of the pressure let-down section. The fourth expression mode pressure profile has e.g. a sinusoidal shape.

In embodiments, at least one of the plurality of predetermined expression mode pressure shapes has a pressure build-up section and a pressure let-down section. For a single period the wave is approximately mirrored around the moment where the amplitude has been reached. Thus, the slope of the pressure build-up section is similar to the slope of the pressure let-down section. The pressure shape can e.g. be convex. Optionally, the pressure shape has a prolonged time period between a pressure let-down section and the pressure build-up section of the next period.

In embodiments, the pump system comprises a user input module for receiving the user input setting, e.g. a touch screen or a button. The user can thus provide the user input settings by manipulating the user input module. The user input module may be configured to generate a user input signal based on the received user input setting, and transmit this user input signal to the control unit.

In embodiments, the pump system comprises a communication terminal configured to receive the user input setting through wireless communication. The communication terminal may be configured to generate a user input signal based on the received user input setting, and transmit this user input signal to the control unit. The communication terminal may e.g. apply any suitable wireless communication technique or protocol, e.g. Bluetooth, 4G, 5G, 6G, or Wi-Fi. The communication terminal can e.g. be configured to communicatively connect to a user equipment device (e.g. a mobile device such as smartphone, tablet, computer, or remote control). The user can provide the user input setting on said user equipment device (e.g. via a mobile application), which is then transmitted to the communication terminal of the pump system. It may also be possible that the communication terminal can e.g. be configured to communicatively connect to a cloud-based system, which in turn can e.g. be configured to communicatively connect to a user equipment device. The user input settings are then inputted by the user on the user equipment device, which transmits the user settings to the cloud-based system, which in turn transmits the user settings to the communication terminal.

In embodiments, the control unit is further configured to control the force unit according to stimulation mode prior to the expression mode. Applying a stimulation mode may be advantageous to start the milk flow. During the stimulation mode, the control unit may be configured to apply a stimulation mode pressure profile. The stimulation mode pressure profile may have a stimulation frequency, a stimulation amplitude, and a stimulation pressure shape. Generally, the stimulation frequency may be relatively high. The stimulation frequency is generally higher than the frequency applied in the expression mode pressure profile later. Optionally, the stimulation pressure shape is the same as the pressure shape to be applied in the expression mode pressure profile later. Optionally, the stimulation pressure shape is different from the pressure shape to be applied in the expression mode pressure profile later.

Optionally, the stimulation mode may be embodied similarly to any of the embodiments described herein for the expression mode, mutatis mutandis.

Optionally, the control unit is configured to receive stimulation user settings. Optionally, the stimulation user settings relate to a stimulation frequency and/or stimulation amplitude. Optionally, the stimulation user settings also relate to a stimulation pressure shape. For example, the stimulation pressure shape may be chosen from a plurality of predetermined stimulation mode pressure shapes. The stimulation mode pressure shapes may e.g. be similar or the same as the expression mode pressure shapes. Optionally, the control unit is configured to receive stimulation user settings simultaneously with the user settings for the expression mode. Optionally, the user settings may relate to a single pressure shape, which is applied to both the stimulation mode and the expression mode (i.e. only having different frequency and/or amplitude). However, it may also possible that the user settings allow the user to change different pressure shapes for the stimulation mode and the expression mode.

In embodiments, the pump system comprises a second pressure outlet configured to be connected to a second breast cup. The pump system is configured to provide a varying pressure to the second breast cup. This allows e.g. for a mother to extract milk from both breast at the same time. The second pressure outlet may be configured similarly as the first pressure outlet, e.g. according to any of the embodiments described herein. For example, the force unit may be configured to control the pressure at the both the pressure outlet and the second pressure outlet. The pressure at the second pressure outlet may e.g. be controlled by the same outlet membrane. It may also be possible that the second pressure outlet comprises a second outlet membrane. The force unit, e.g. by means of the stepper motor, may be configured to move the second outlet membrane, e.g. simultaneously with the outlet membrane. The control unit may e.g. be configured to generate the same expression mode pressure profile in both breast cups. Optionally, the pressure variations generated by the pressure outlet and the second pressure outlet are in phase with each other. Optionally, the pressure variations generated by the pressure outlet and the second pressure outlet are in counter-phase with each other.

It is noted, however, that in embodiments it may be possible to generate varying pressure at two breast cups with a single pressure outlet. In those embodiments, a fluidic connection may branch off into two fluidic connections, each connected to one of the breast cups.

In embodiments, the pump system is configured to be portable during use. Below some examples possible characteristics of a pump system being portable during use are elaborated on. It is noted that such characteristics can e.g. be made possible by various embodiments of the invention. In particular, the use of a stepper motor in the force unit may allow smaller dimensions, and/or lower weight, and/or less energy consumption (allowing the use of a battery as power source) in comparison to conventional systems, while at the same time enabling more controllability of the pressure profile.

Advantageously, the pump system as explained herein is able to reach combinations of frequency and (negative) pressure that the large hospital breast pumps can achieve, while being portable. Conventional breast pumps can be divided in two categories in terms of portability. Larger systems, e.g. used in hospitals and sometimes rented out by (social) insurance companies, can provide controllability, but are obviously not portable. This limits their usability for the mother. Conventional portable systems on the other hand, use pumping systems that are less controllable and thus have several disadvantages on that aspect. The invention can, in embodiments, provide the solutions for these problems.

For example, the dimensions of the pump system may be small enough to be comfortable to be carried around during use. For example, the pump system may comprise a pump housing comprising at least the pressure outlet, the force unit, and the control unit, wherein the pump housing is e.g. smaller than 30 cm in the largest direction, e.g. smaller than 20 cm, e.g. 15 cm or smaller. For example, the pump housing may be 25x20x15cm, e.g. smaller than 20x15x10 cm, e.g. 15x12x6 cm or smaller. The pump housing may e.g. be generally beam-shaped or cuboid-shaped (although it will be understood that deviations in this shape are possible, e.g. for connectors, pressure outlet(s), buttons, rounded edges, etc.).

For example, the weight of the pump system may be low enough to comforfable enough to be carried around during use. For example, the weight of the pump system may be less than 2 kg, e.g. less than 1.5 kg, e.g. less than 1 kg, e.g. 700 g or less.

For example, the pump system may comprise a battery, wherein the battery is configured to provide (electrical) power to the force unit. Optionally, the battery is configured to be rechargeable, e.g. using a USB connection, e.g. a USB-C or a mini-USB.

For example, the pump system comprises a carrying element. The carrying element allows the user to carry the pump system during use. For example, the carrying element can include a clip allowing to attach the pump system to clothing, e.g. to pants. For example, the carrying element include be a strap, allowing to carry the pump system while supported by the shoulders (e.g. like a purse). The pump housing may e.g. have connection loops for connecting the strap. For example, the carrying element can include a handle. Multiple carrying elements, e.g. combining two or more of the above, are possible as well.

In embodiments, the force unit is configured to provide varying pressure at the pressure outlet by changing a volume. For example, the force unit may be configured to move one or more outlet membranes. For example, the force unit may be configured to move a piston.

In embodiments, the force unit comprises a stepper motor, wherein e.g. the stepper motor is configured to move the outlet membrane.

In embodiments, the pump system comprises a motor shaft connected to the stepper motor, wherein the stepper motor is configured to cause linear movement of the motor shaft, wherein the motor shaft is configured to cause linear movement of the at least one membrane. For example, the stepper motor may be configured to be moved in a rotational direction with each step. The rotational movement can be converted to a linear movement. For example, the stepper motor may comprise or move a toothed wheel in the rotational direction. The motor shaft may be toothed, wherein the teeth of the motor shaft interact with the teeth of the wheel. The wheel is configured to cause linear movement of the motor shaft by rotating. For example, the wheel may act as pinion and the motor shaft may act as rack. Since the steps of the stepper motor can be controlled accurately, the movement of the motor shaft can also be controlled accurately.

In embodiments, the pump system comprises the motor shaft connected to the stepper motor, wherein the motor shaft is rigidly connected to the outlet membrane. Thus, the connection from the motor shaft (or stepper motor itself) to the outlet membrane is rigid, and not flexible. Thus, the connection is not done by means of a belt or similar flexible element. The rigid connection ensures that the accurate movement of the stepper motor results in accurate (and known/predictable) movement of the outlet membrane. This may also allow to determine the transfer function (how movement of the stepper motor affects pressure at the nipple) more accurately, since there is a well-defined relationship between the movement of the stepper motor and the membrane.

In embodiments, the pump system further comprises a connection element connected to the motor shaft and to the at least one outlet membrane. Optionally, the connection element is a rigid element. The connection element transfers movement of the motor shaft to the membrane.

In embodiments, the pump system comprises a clamping element (e.g. clamping plate) and a clamping counter-element (e.g. clamping counter-plate) configured to clamp the membrane between them, wherein the clamping element and clamping counter-element are configured to be moved by movement of the motor shaft.

In embodiments, the pump system comprises a pressure space (e.g. in a pressure space housing), wherein the volume of the pressure space is determined by the at least one outlet membrane. The pressure outlet is fluidly connected to the pressure space. The outlet membrane is configured to be deformed and/or moved (when the stepper motor is moves). This changes the volume of the pressure space, which allows to generate varying pressure at the pressure outlet.

Optionally, the pressure space (and/or pressure space housing, and/or the outlet membrane when in a end position) has a depth, a width, and a height, wherein the height is in a direction parallel to the longitudinal axis of the motor shaft, and the depth and width are both perpendicular said longitudinal axis of the motor shaft (and thus also to the height), and the depth and width are perpendicular to each other. At least one of the depth and width is larger than the height, e.g. at least 2.5 times larger, e.g. at least 4 times larger. This allows for relatively large changes in volume of the pressure space for relatively small movement of the motor shaft, which in turn allows for a compact design.

In embodiments wherein the pump system comprises two outlet membranes, the connection element may comprise a first connector connected to the first outlet membrane, and a second connecter connected to the second membrane. The first and second connector may be symmetric to each other, e.g. in view of a symmetry line that extends through a longitudinal centre axis of the motor shaft.

In embodiments, the pump system comprises a home position sensor configured to detect whether the stepper motor is in a home position, and to generate a home position signal, wherein the control unit is configured to receive the home position signal. The home position can e.g. be a rest position or zero position of the stepper motor (and the motor shaft). The control unit may be configured to determine the step sequence as steps relative to the home position. Optionally, the home position sensor is a contact sensor. Optionally, a sensor pusher is attached to the motor shaft and configured to contact the contact sensor. Optionally, the control unit may be configured to control the stepper motor to pass the home position in each period of the expression mode pressure profile.

In embodiments, the control unit is configured to apply a feed forward control and/or open loop control for controlling the force unit. Thus, the control unit does not take into account any feedback, meaning it is not necessary to provide additional sensors or similar components. This is advantageous, as it lowers the cost and complexity in comparison to systems using one or more feedback signals (e.g. from pressure sensors). In particular when a stepper motor is used, the position of the motor shaft and thus the outlet membrane(s) can easily be determined from the number of steps that the stepper motor has been moved.

In embodiments, the pump system comprises a pressure sensor configured to measure a pressure at the at least one pressure outlet, generate a pressure signal, and transmit the pressure signal to the control unit. In embodiments, the pump system comprises a pressure sensor configured to measure a pressure at breast cup, e.g. in a nipple receiving portion, generate a pressure signal, and transmit the pressure signal to the control unit. In these embodiments, a feedback signal is provided by means of the pressure signal, which the control unit can be configured to take into account when controlling the force unit.

In embodiments, the invention may relate to a breast pump system for extracting milk from a nipple of a human breast. The breast pump system may comprise a pump system according to any of the embodiments described herein. The breast pump system may optionally further comprise at least one breast cup.

Optionally, the breast pump system comprises a connection tube configured to be connected to the breast cup at a first end and to the pressure outlet at a second end. The connection tube fluidly connects the pressure outlet of the pump system to the breast cup. The connection tube can e.g. be made from a flexible material, e.g. a polymer. The connection tube can e.g. be transparent.

Optionally, the breast pump comprises an incorporated/integrated system comprising the pump system and the breast cup. In these embodiments it may be possible to fluidly connect the pressure outlet to the breast cup without a connection tube. Optionally, the pump system and/or breast cup are releasable connected to the incorporated system. This allows to remove them, e.g. for cleaning.

In embodiments, the breast pump system, further comprises a milk container configured to receive milk extracted from the breast, wherein optionally the milk container is incorporated/integrated in the breast cup.

In embodiments, the system further comprises a mobile application. The mobile application can e.g. be configured to be executed on a user equipment device (e.g. a smartphone, tablet, smartwatch, computer). The mobile application is configured to be communicatively connected to the control unit of the pump system (e.g. via a communication terminal). The mobile application can e.g. be configured to transmit the user settings to the control unit, wherein the user can provide the user settings on the user equipment device. The mobile application can e.g. be configured to present data to the user. The mobile application can e.g. be executed with non-transitory computer readable instructions.

In embodiments, the system further comprises a cloud-based infrastructure configured to be communicatively connected to control unit of the pump system and/or the local user equipment device, and e.g. comprising at least one server having a processing unit. The cloud-based infrastructure may e.g. be configured to communicate via an internet communication, e.g. WI-FI, 3G, 4G, or 5G.

The invention further relates to one or more methods. Although the methods can be performed with the pump system according to the invention; neither the pump system, nor the method is limited thereto. Features explained herein with reference to the pump system have the same meaning with respect to the method unless explicitly defined otherwise. Features explained with reference to the pump system can be applied mutatis mutandis to the method to achieve the similar advantages, and vice versa.

One or more objects of the invention can be achieved with a method for extracting milk from a human breast, wherein the method comprises a step of using a pump system or breast pump according to any of the embodiments described herein. Optionally, the method further comprises a step of using the force unit of the pump system to generate a varying pressure at the nipple.

One or more objects of the invention can be achieved with a method for extracting milk from a human breast, the method optionally comprising a step of using a pump system according to any of the embodiments described herein, to subject the breast to the first expression mode pressure profile and/or the expression mode second pressure profile. Optionally, the method comprises a step of receiving user input settings, and determining an expression mode pressure profile based thereon.

In embodiments, the invention relates to (non-transitory) computer readable instructions, configured to cause a control unit of a pump system (e.g. according to any of the embodiments described herein) to perform a method according to the any of the embodiments described herein.

Exemplary embodiments of the invention are described using the figures. It is to be understood that these figures merely serve as example of how the invention can be implemented and are in no way intended to be construed as limiting for the scope of the invention and the claims. Like features are indicated by like reference numerals along the figures. In the figures:
Fig. 1a-1c schematically illustrate a housing of a pump system for a breast pump;
Fig. 2a-2d schematically illustrate the pump system;
Fig. 3a-3e schematically illustrate expression mode pressure profiles with different pressure shape,
Fig. 4 schematically illustrates the working principle of a control unit of the pump system.

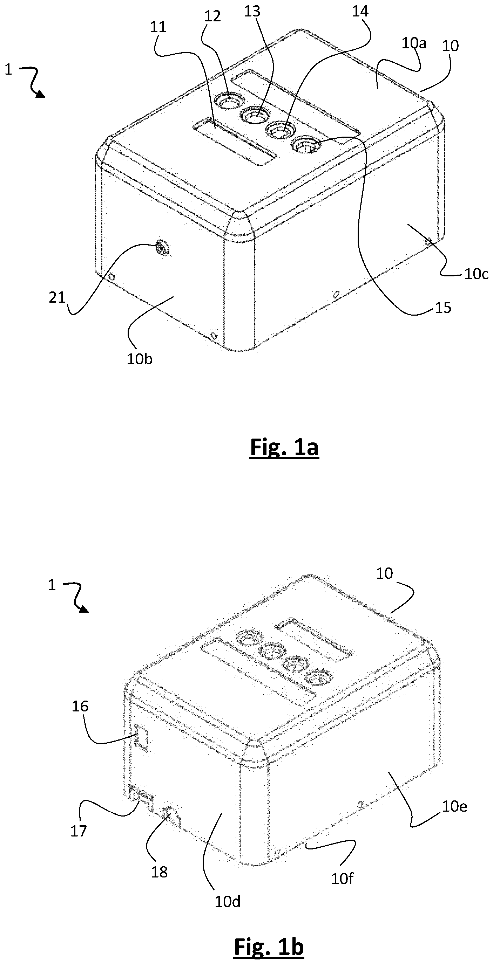

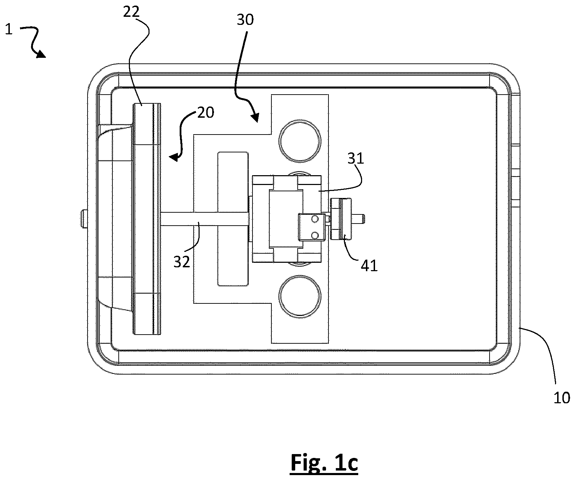

Fig. 1a-1c schematically illustrate a housing 10 of a pump system 30 for a breast pump 1. Fig. 1a illustrates a top surface 10a, a first side surface 10b, and a second side surface 10c. Fig. 1b shows a third side surface 10d, a fourth side surface 10e, and bottom surface 10f. In fig. 1c the bottom surface 10f is not shown to make the pump system 30 visible.

A pressure outlet 21 is provided in the first side surface 10b. A tube (not shown) can be connected to the pressure outlet 21 by arranging the pressure outlet 21 in an open end of the tube. An opposite end of the tube can be connected to a breast cup. The breast cup can be placed on a nipple of a human breast. The pump system 30 can provide a varying pressure at the pressure outlet 21, which is transmitted to the breast cup via the tube. The breast cup can in turn subject the nipple to the varying pressure, which will result in the extraction of milk from the breast (of course on the condition that the breast is from a mother with milk production).

Fig. 1a further illustrates schematically that screen 11 can be provided in the housing 10. The screen 10 can present information to the user (e.g. the mother). Such information can e.g. relate to whether the pump system 30 is turned on/active, settings of the pump system 30, battery status, etc. However, other ways of transmitting information to the user can also be envisaged, e.g. by means of (LED) lighting. Several buttons 12, 13, 14, 15 can allow the user to provide user settings to the pump system. User settings can e.g. relate to activating the pump system 30, but also to desired operating settings of the pump system 30, e.g. relating to a pressure profile created in the varying pressure. Fig. 1b illustrates several ports 16, 17, 18 provided in the housing, which can e.g. be used for charging the battery and/or for data connections.

Fig. 1a-1c illustrate that the housing 10 has a substantially beam-shaped shape, albeit that some corners are rounded. The bottom surface 10f can be used to arrange the housing 10 on a supporting surface such as a table. However, the pump system 30 (and thus the housing 10) is also portable during use. This allows a user to walk around and do normal activities while pumping. The breast pump 1 can e.g. have a weight of 700g, and dimensions of 15x12x6cm.

Fig. 1c illustrates the pump system 30. In the shown example the pump system 30 comprises a stepper motor 31. The stepper motor 31 moves in steps, which allows good controllability. The rotation of the stepper motor 31 at each step is transferred to a motor shaft 32, which is moved in linear direction at each step. Although not explicitly shown in the figures, it will be understood that the stepper motor 31 and the motor shaft 32 comprise teeth that interact with each other to convert the rotational movement of the stepper motor 31 into the linear movement of the motor shaft 32.

The linear movement of the motor shaft 32 is in a direction parallel to the longitudinal center axis of said motor shaft 32. In the view shown in fig. 1c, said movement is to the lefthand side and the righthand side. In the situation shown in fig. 1c, the motor shaft 32 is in a home position, which is the position most to the lefthand side. It can be seen that space is available in the housing 10 for the motor shaft 32 to move to the righthand side. The motor shaft 32 is connected to a pressure space 20 to which also the pressure outlet 21 is connected. The pressure space 20 is defined within a pressure space housing 22.

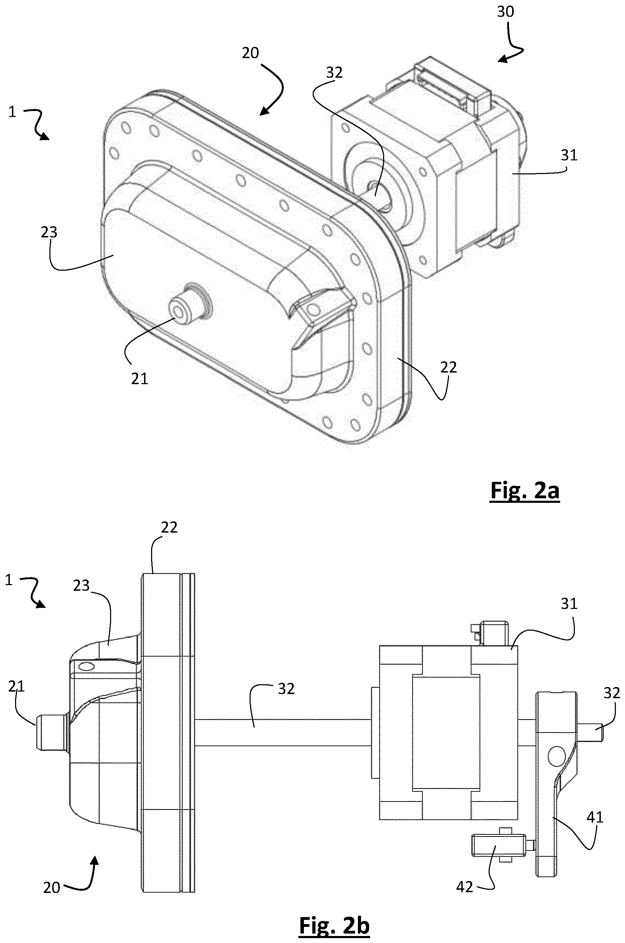

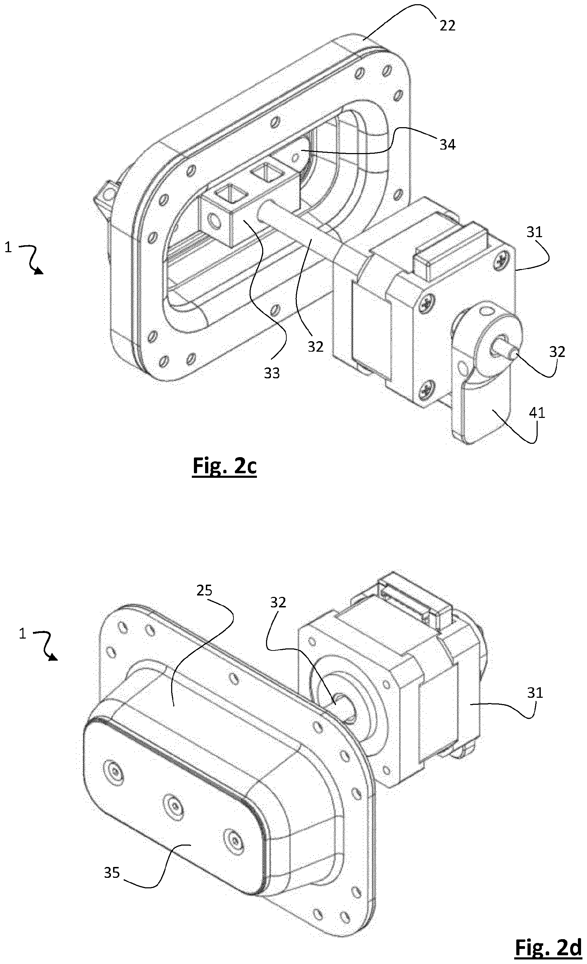

Fig. 2a-2d schematically illustrate the pump system 30. Fig. 2a shows an isometric view, fig. 2b shows a side view, fig 2c shows another view, and fig. 2d shows the pump system 30 without the pressure space housing 22.

In fig. 2c it can be seen that the motor shaft 32 is at an end section is connected to a connection element 33. The connection element 33 in turn is connected to clamping plate 34. Clamping plate 34 is connected to a clamping counter-plate 35 (fig. 2d). Between the clamping plate 34 and the clamping counter-plate 35, a membrane 25 is clamped. The movement of the motor shaft 32 will as such be transmitted to the membrane 25 via the connection element 33, clamping plate 34, and clamping counter-plate 35.

The membrane 25 is arranged in the pressure space housing 22. The pressure housing 22 comprise a membrane moving section 23, which has a shape adapted to the membrane 25. The space in the membrane moving section 23 between the membrane 25 and the pressure outlet 21 is sealed by the membrane 25. Thus, the available space for air in that space is defined by the position of the membrane 25. When the membrane 25 is moved by the motor shaft 32, said available space for air become larger or smaller. This affects the pressure in said space and at the pressure outlet 21, such that the varying pressure can be achieved.

The membrane 25 is made of a flexible material, e.g. a polymer. This allows to deform the membrane 25 during the movement of the motor shaft 32. Such deformation can be advantageous to change the space available, while also keeping said space sealed to avoid air coming in or escaping.

The connections between the stepper motor 31 and the membrane 25 are made by rigid elements 32, 33, 34, 35, which do not deform (at least not noticeable under the stress applied during normal functioning of the system 30). The rigid connections allow an accurate movement of the membrane 25, since the movement of the motor shaft 32 is practically equal to the movement of the membrane 25. In addition, not many intermediate components are required (in contradiction to known systems which use e.g. belts or various gears between the motor and the membrane), which allows to make a compact motor system. A compacter motor system allows for a smaller housing, which is beneficial for the portability during use.

The pressure space 20 is defined by a depth (in fig. 2b in vertical direction), a width (in fig. 2b in the direction perpendicular to the paper), and a height (in fig. 2b in horizontal direction). The width is larger than the height, at least 2.5 times larger. This allows for relatively large changes in volume of the pressure space for relatively small movement of the motor shaft 32, which in turn allows for a compact design.

In fig. 2b a home position sensor 42 is visible. The home position sensor 42 is configured the detect when the stepper motor 31 and thus the motor shaft 32 is in a home position (which is the case in the shown figures). In the shown example a sensor pusher 41 is connected to the motor shaft 32 and moves along with the motor shaft 32. The sensor pusher 42 has a protrusion that extends away from the motor shaft 32, in fig. 2b in the vertically downward direction. The protrusion of the sensor pusher 42 comes into contact with (and presses) the home position sensor 42 when the stepper motor 31 and motor shaft 32 are in the home position. The home position sensor 42 is a contact sensor in this case, which is configured to generate a home position signal which indicates that the protrusions of the sensor pusher presses the home position sensor 42. From the home position signal, it can be detected that the stepper motor 31 is in the home position.

The pump system 30 as shown in the previous figures is advantageous for various reasons. Firstly, the design can be very compact and lightweight, making it possible to carry the pump system 30 around during use. This is advantageous, as it allows the mother to perform other activities at while pumping milk. The housing 10 can e.g. be provided with a carrying element, e.g. a handle or a connection loop for attaching a strap. With such strap, the mother can carry the housing around like a purse. Secondly, the stepper motor 31 and the way it is connected to the membrane 25 allow for great controllability. This controllability allows in particular to give the user the option between different pressure profiles. In this context, a pressure profile can e.g. be characterized by a frequency, an amplitude, and a pressure shape. Whereas conventional (portable) pump systems only allow to adapt the frequency and amplitude, the system as shown in the figures also allows to adapt the pressure shape.

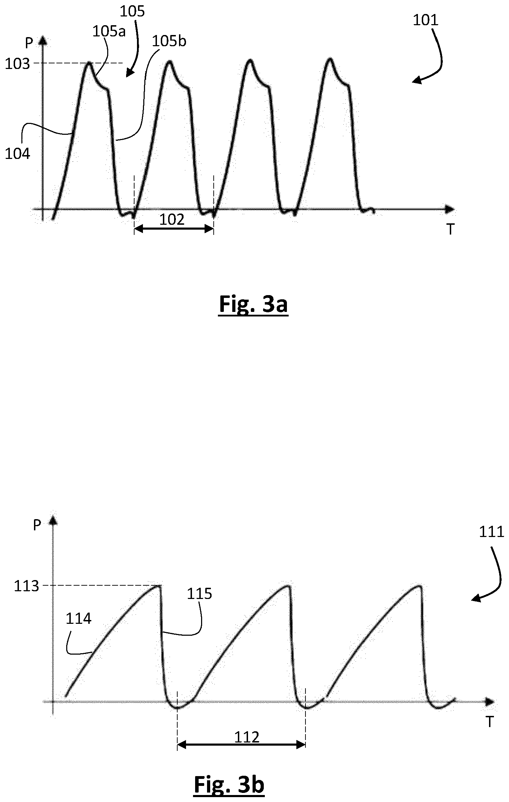

Examples of pressure profiles with different pressure shapes that are possible with the pump system are shown in fig. 3a-3e. These figures show a graph of the varying pressure, as exerted onto the breast. Thus, the pressure can be measured at the nipple, e.g. within a nipple receiving portion of the breast cup. The horizontal axes in these figures illustrate the time (T), the vertical axes illustrate the negative pressure (P). Although the pressure in generally depicted as positive in these graphs, it will be understood that in general a negative pressure is exerted onto the breast to emulate the sucking action of the child. Therefore, the pressure can be considered to be the opposite of what is shown in these graphs.

The pressure profiles shown in the fig. 3a-3e are expression mode pressure profiles. They are configured to be used during an expression mode of the breast pump 1. It is common practice to first apply a stimulation mode pressure profile to the breast to activate the flow of milk. The stimulation mode pressure profile is usually higher in frequency. Then, an expression mode pressure profile is applied to extract the milk from the breast. Optionally, the stimulation mode pressure profile may apply the same pressure shape as the shown stimulation mode pressure profile, but at higher frequency.

Fig. 3a illustrates a first expression mode profile 101, which is a periodic function having a period 102 and an amplitude 103. The frequency of the periodic function is the inverse of the period. The function has a pressure build-up section 104 and a pressure let-down section 105. The pressure let-down section has a first part 105a and a second part 105b, with different slope. In the first part 105a, pressure is released slower than in the second part 105b. In the second part 105b, the slope is similar in absolute terms to the slope in the rising part 104. The pressure let-down section occupies more than 50% of the period (the pressure shape in time) in this example.

Fig. 3b illustrates a second expression mode profile 111, which is a periodic function having a period 112 and an amplitude 113. The frequency of the periodic function is the inverse of the period. The function has a pressure build-up section 114 and a pressure let-down section 115. The pressure let-down section 115 has a steep slope as the pressure is released quickly. The pressure build-up section 114 has a slope that is less steep, as pressure is built up gradually. The pressure build-up section occupies the majority of the period (the pressure shape in time) in this example, even more than 80%.

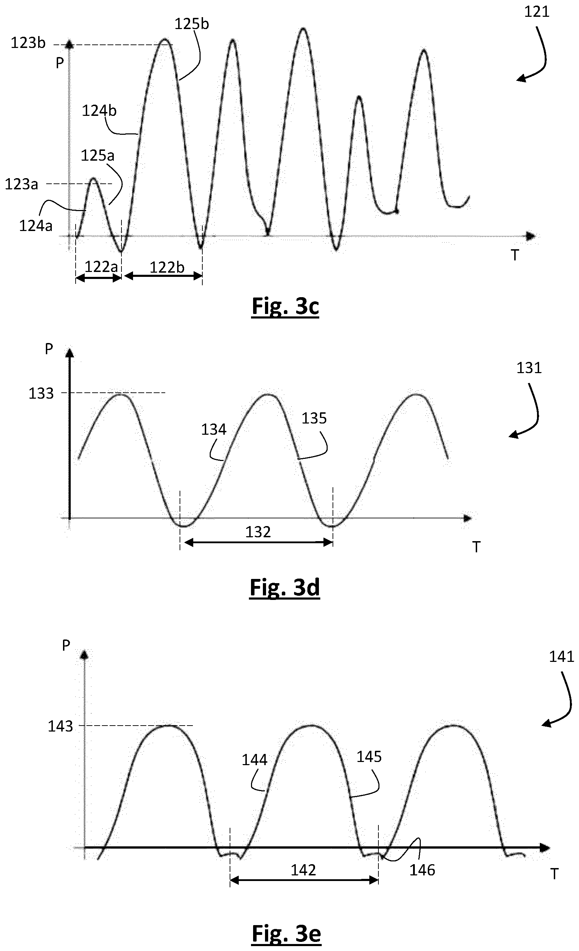

Fig. 3c illustrates a third expression mode profile 121, which is a non-periodic function having a periods 122a, 122b, and amplitudes 123a, 123b that vary. Such expression profiles emulate the sucking behavior of the child, since a child will not exert a perfectly periodic pressure profile. When a user would select such pressure profile in the user settings, the chosen amplitude and frequency may be the average maximal amplitude and average frequency, respectively. Each single wave has a period 122a, 122; an amplitude 123a, 123b, a pressure build-up section 124a, 124b, and a pressure let-down section 125a, 125b. For a single wave it can be seen that the wave is approximately mirrored around the moment where the amplitude 123a, 123b has been reached. Thus, the slope of the respective pressure build-up section s 124a, 124b is similar to the slope of the respective pressure let-down section 125a, 125b.

Fig. 3d illustrates a fourth expression mode profile 131, which is a periodic function having a period 132 (inverse of frequency) and an amplitude 133. The pressure profile has a pressure build-up section 134 and a pressure let-down section 135. For a single period it can be seen that the wave is approximately mirrored around the moment where the amplitude 133 has been reached. Thus, the slope of the pressure build-up section 134 is similar to the slope of the pressure let-down section 135. The fourth expression mode pressure profile 131 has a sinusoidal shape.

Fig. 3e illustrates a fifth expression mode profile 141, which is a periodic function having a period 142 (inverse of frequency) and an amplitude 143. The pressure profile has a pressure build-up section 144 and a pressure let-down section 145. For a single period it can be seen that the wave is approximately mirrored around the moment where the amplitude 143 has been reached. Thus, the slope of the pressure build-up section 144 is similar to the slope of the pressure let-down section 145. The pressure shape is convex., the pressure shape has a prolonged time period 146 between a pressure let-down section 145 and the pressure build-up section 144 of the next period.

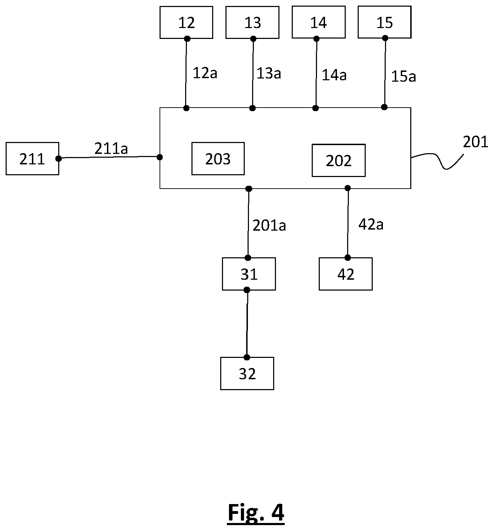

Fig. 4 schematically illustrates the working principle of a control unit 201. The control unit 201 can be arranged in the housing as shown in the previous figures, and is configured to control the pump system by controlling the stepper motor 31.

The control unit 201 can be embodied according to any suitable embodiment, as many types of control units are known. The control unit 201 comprises a memory 202 for storing data, e.g. historic data, computer-readable instructions, user input settings, control settings for the stepper motor 31, pressure profiles, pressure shapes, etc. The control unit 201 comprises a processing unit for making processing steps and/or calculations.

The control unit 201 is configured to receive user input settings. The user can provide these user input settings in different ways. For example, the user can push on one of the buttons 12, 13, 14, 15 to generate a respective user input signal 12a, 13a, 14a, 15a that is transmitted to the control unit 101. The buttons 12, 13, 14, 15 can be part of a user input module. It is also possible that the control unit 201 comprises a communication terminal 201.1 configured to receive user input settings via a wireless communication signal 211a. The user can e.g. provide the user input settings on a mobile application 211, which transmits the user input settings to the control unit 201. Although fig. 4 illustrates the wireless communication signal 201.1 directly to the control unit 201, in practice it may be possible that a cloud-based system is used as well, arranged functionally between the mobile application 211 and the control unit 201.

The user input settings may relate to a desired frequency and amplitude (i.e. maximal pressure) that the user desires. In addition or alternatively, the user input settings may relate to a pressure shape that the user desires. The user can e.g. choose between a plurality of predetermined expression mode pressure shapes. Said plurality can e.g. include two or more of the pressure shapes shown in fig. 3a-3e. It has been found that some pressure shapes work well for some mothers, while other pressure shapes work well for other mothers. Even for a single mother, the optimal pressure shape may depend on changing factors, and thus change as well. Allowing the user to choose between different pressure shapes, ensures that a suitable pressure shape can be found.

The control unit 201 determines an expression mode pressure profile based on the user input settings. This can e.g. be achieved by scaling a baseline pressure profile for the chosen pressure shape, based on the chosen frequency and/or amplitude. The control unit 201 then generates a control signal 201a to control the steps of the stepper motor, based on the determined expression mode pressure profile. The control signal 201a ensures that the stepper motor 31 is moved in a step sequence from a begin step to a maximal step, and back. The step sequence depends on the pressure shape, the maximal step depends on the amplitude, and the speed at which the step sequence is performed depends on the frequency.

It will be understood that even when the same frequency and same amplitude would be chosen for two different pressure shapes, this would result in a different step sequence. Within any step sequence, the control unit may have freedom to determine the size of the step, the speed at which one or more steps are taken, the direction of the steps, and/or the time period between subsequent steps. Two different step sequences may hence also differ from each other in the size of the steps, and/or the speed in which steps are taken, and/or a time period between subsequent steps, and/or the direction of the steps.

The desired expression mode pressure profile reflects the pressure at the breast. When determining a step sequence based thereon, the control unit 201 may take into account a transfer function (or the inverse thereof). The transfer functions reflects how movement of the motor shaft 32 affects the pressure at the breast. This may be dependent on the breast cup, but it may also be possible to implement a generic model if the type of breast cup is not known.

The control unit 201 is further configured to receive the home position signal 42a from the home positions sensors 42. Based on this it can be confirmed that the stepper motor 31 is in the home position. Any steps taken in the step sequence, can e.g. be determined relative to the home position. The position of the stepper motor 31, the motor shaft 32, and as such the membrane can be known accurately by keeping track of the steps taken. Accurate control of the pressure at the breast can be achieved, without the need for feedback by additional sensors or similar.

As required, detailed embodiments of the present invention are described herein; however, it is to be understood that the disclosed embodiments are merely examples of the invention, which may be embodied in various ways. Therefore, specific structural and functional details disclosed herein are not to be construed as limiting, but merely as a basis for the claims and as a representative basis for teaching those skilled in the art to practice the present invention in various ways in virtually any suitable detailed structure. Not all of the objectives described need be achieved with particular embodiments.

Furthermore, the terms and expressions used herein are not intended to limit the invention, but to provide an understandable description of the invention. The words "a", "an", or "one" used herein mean one or more than one, unless otherwise indicated. The terms "a multiple of", "a plurality" or "several" mean two or more than two. The words "comprise", "include", "contain" and "have" have an open meaning and do not exclude the presence of additional elements. Reference numerals in the claims should not be construed as limiting the invention.

The mere fact that certain technical features are described in different dependent claims still allows the possibility that a combination of these technical measures can be used advantageously.

A single processor or other unit can perform the functions of various components mentioned in the description and claims, e.g. of processing units or control units, or the functionality of a single processing unit or control unit described herein can in practice be distributed over multiple components, optionally physically separated of each other. Any communication between components can be wired or wireless by known methods.

The actions performed by the control unit can be implemented as a program, for example computer program, software application, or the like. The program can be executed using computer readable instructions. The program may include a subroutine, a function, a procedure, an object method, an object implementation, an executable application, a source code, an object code, a shared library / dynamic load library and / or other set of instructions designed for execution on a computer system.

A computer program or computer-readable instructions can be stored and / or distributed on a suitable medium, such as an optical storage medium or a solid-state medium supplied with or as part of other hardware, but can also be distributed in other forms, such as via internet or other wired or wireless telecommunication systems.

## Claims

1. A pump system for a breast pump for extracting milk from a human breast, the pump system comprising,
• at least one pressure outlet comprising at least one outlet membrane, wherein the pressure outlet is configured to be fluidly connected to a breast cup configured to be arranged on the breast,
• a force unit configured to move the at least one outlet membrane for controlling the pressure at the air outlet, wherein the force unit comprises a stepper motor configured to be moved in steps, and configured to transmit a movement to the outlet membrane with each step,
• a control unit configured to control the force unit, wherein the control unit is configured to
• receive user input settings, wherein the user input settings relate to a frequency, an amplitude, and a pressure shape chosen from a plurality of predetermined expression mode pressure shapes,
• determine an expression mode pressure profile based on the user input settings, and control the force unit based on the expression mode pressure profile, wherein:
• for a first expression mode pressure profile based on user settings with a first pressure shape, and a second expression mode pressure profile based on user settings with a second pressure shape, the pressure at the air outlet in function of time differ from each other in more and/or other ways than being scaled in amplitude and/or time.

2. The pump system according to claim 1, wherein the control unit is configured to determine a step sequence for the stepper motor based on the expression mode pressure profile, wherein,
• the first expression mode pressure profile and the second expression mode pressure profile are both based on user settings with a first frequency, a first amplitude;
• for the first expression mode pressure profile the control unit is configured determine a first step sequence for moving the stepper motor from a begin step to a first maximal step and back within a first time period,
• for the second expression mode pressure profile the control unit is configured to determine a second step sequence for moving the stepper motor from the begin step to the first maximal step and back within the first time period,
wherein the first step sequence differs from the second step sequence in the size of the steps, and/or the speed in which steps are taken, and/or a time period between subsequent steps, and/or a direction of each of the steps.

3. The pump system according to claim 1, wherein the stepper motor is configured to take a plurality of different step sizes.

4. The pump system according to any of the preceding claims, wherein the plurality of predetermined expression mode pressure shapes comprises at least four different expression mode pressure shapes, each configured to result in mutually different step sequences at equal amplitude and frequency.

5. The pump system according to any of the preceding claims, wherein the first pressure shape is a sinusoidal shape, and wherein
the second pressure shape is a shape determined by a polynomial having at least five factors, e.g. at least eight factors.

6. The pump system according to any of the preceding claims, wherein the first pressure shape has a pressure build-up section and a pressure let-down section, wherein
• the pressure let-down section occupies at least 40% of the first pressure shape in function of time; and/or
• wherein the slope of the pressure let-down section is equal to the slope of the pressure build-up section; and/or
• wherein the pressure build-up section and the pressure let-down section are mirrored to each other relative to a mirror line which extends through a peak.

7. The pump system according to any of the preceding claims, wherein the control unit is configured to enable, within each expression mode, to adjust the frequency and/or the amplitude.

8. The pump system according to any of the preceding claims, wherein the pump system is configured to be portable during use.

9. The pump system according to any of the preceding claims, comprising a motor shaft connected to the stepper motor, wherein the stepper motor is configured to cause linear movement of the motor shaft, wherein the motor shaft is configured to cause linear movement of the at least one membrane.

10. The pump system according to any of the preceding claims, comprising a motor shaft connected to the stepper motor, wherein the motor shaft is rigidly connected to the outlet membrane.

11. The pump system according to any of the preceding claims, wherein the control unit is configured to apply a feed forward control and/or open loop control for controlling the force unit.

12. A breast pump system for extracting milk from a nipple of a human breast, comprising a pump system according to any of the preceding claims, further comprising at least one breast cup and a connection tube configured to be connected to the breast cup at a first end and to the pressure outlet at a second end.

13. A system according to any of the preceding claims, comprising a mobile application, wherein the mobile application is be configured to
• be executed on a user equipment device
• be communicatively connected to the control unit of the pump system
• transmit the user settings to the control unit, wherein the user can provide the user settings on the user equipment device.

14. A method for extracting milk from a human breast, the method comprising a step of using a pump system according to any of the preceding claims to subject the breast to the first expression mode pressure profile and/or the expression mode second pressure profile.

15. Non-transitory computer readable instructions, configured to cause a control unit of the pump system to perform a method according to the preceding claim.
